# FASCICULE DE BREVET EUROPEEN

(11) **EP 2 856 144 B1**
(45) Date de publication et mention de la délivrance du brevet: **08.01.2020**
(21) Numéro de dépôt: 12731531.5
(22) Date de dépôt: 04.06.2012
(51) Int. Cl.: G01N 33/00, G01N 33/28, G01N 33/22, G01N 30/16

(54) **ANALYSEUR DE FLUIDE COMPORTANT UNE SUBSTANCE INFLAMMABLE ET PROCÉDÉ CORRESPONDANT**
VORRICHTUNG ZUR FLÜSSIGKEITSANALYSE MIT EINEM BRENNBAREN MEDIUM SOWIE ENTSPRECHENDES VERFAHREN
FLUID ANALYSER COMPRISING A FLAMMABLE SUBSTANCE AND CORRESPONDING METHOD

(43) Date de publication de la demande: 08.04.2015
(73) Titulaire: Apix Analytics, 38000 Grenoble (FR); Total SA, 92400 Courbevoie (FR); EIF-Astute, 93100 Montreuil sous Bois (FR)
(72) Inventeur: ANDREUCCI, Phillipe, F-38430 Moirans (FR); COLINET, Eric, F-38000 Grenoble (FR); CORIC, Philippe, F-44700 Orvault (FR); DE WIT, Jean-Claude, F-92500 Rueil Malmaison (FR); PUGET, Pierre, F-38330 Saint-Isnier (FR)
(74) Mandataire: Lavoix
(86) Numéro de dépôt international: PCT/FR2012/051241
(87) Numéro de publication internationale: WO 2013/182758

(56) Documents cités:
- FR-A1- 2 812 395
- US-A- 5 231 865
- US-A- 5 331 845
- US-A1- 2003 049 854
- US-A1- 2005 063 865

## Description

La présente invention concerne un analyseur du type pour analyser un fluide comportant au moins une substance à analyser et au moins une substance inflammable, l'analyseur comprenant :
- une source de gaz pour fournir un flux de gaz diluteur,
- un injecteur pour introduire des échantillons du fluide dans le flux de gaz diluteur et produire un flux gazeux, et
- un détecteur pour analyser le flux gazeux.

L'invention concerne également un procédé pour analyser un fluide comportant au moins une substance à analyser, ou « analyte », et au moins une substance inflammable, qui peut être l'analyte ou une autre substance.

L'invention est plus particulièrement adaptée à l'analyse de fluides présentant un risque d'explosion, ou à une analyse réalisée dans une atmosphère potentiellement explosive. L'invention est par exemple adaptée pour les analyses réalisées dans l'industrie pétrolière, la chimie et la pétrochimie.

Une atmosphère explosible, ou « ATEX », est un mélange avec l'air, dans les conditions atmosphériques, de substances inflammables, par exemple sous forme de gaz (méthane, butane, propane, hydrogène...) ou de vapeurs (sulfure de carbone, alcool éthylique, oxyde d'éthylène, acétone...), dans lequel, après inflammation, la combustion se propage à l'ensemble du mélange non brûlé.

Le fluide à analyser peut être liquide dans les conditions dans lesquelles il est prélevé, mais se vaporise dans le gaz diluteur.

Le mélange peut devenir explosif dès que la concentration de la substance inflammable se situe au-dessus d'une Limite Inférieure d'Explosivité, ou « LIE », qui est la concentration minimale de la substance inflammable dans le mélange au-dessus de laquelle le mélange peut être enflammé. La LIE, souvent exprimée en % volumique de la substance inflammable dans l'air, est de l'ordre de 1% ou de quelques % pour de nombreux analytes, et d'environ 0,5% pour les plus inflammables d'entre eux.

US 2005/063865 A1 décrit un analyseur et un procédé selon le préambule des revendications 1 et 11, respectivement. US 5 231 865 A divulgue la dilution des fluides réactifs contenant des particules avec un fluide diluant inerte. FR 2 812 395 A1 divulgue une solution "piège" pour des éléments chimiquement réactifs.

Dans le domaine des analyseurs du type précité, il est connu, pour éviter le risque d'explosion, d'utiliser un gaz diluteur sensiblement dépourvu d'oxygène, par exemple de l'azote obtenu par distillation cryogénique, ou de l'hydrogène, ou encore des mélanges spéciaux non susceptibles de s'enflammer au contact des analytes. Une telle méthode présente l'avantage que l'inflammation du mélange du gaz diluteur avec le fluide à analyser est impossible quel que soit la substance inflammable du fluide à analyser et quelle que soit la masse des échantillons de fluide à analyser.

En revanche, il est nécessaire de disposer d'une source de gaz diluteur, ou gaz de dilution, sensiblement dépourvu d'oxygène, lequel présente en général un coût unitaire non négligeable. En outre, si le gaz diluteur sensiblement dépourvu d'oxygène comporte lui-même une substance inflammable, telle que l'hydrogène, le mélange à évacuer en sortie de l'analyseur présente lui-même un risque d'explosion en cas de contact avec l'air ou plus généralement avec un comburant.

Un but de l'invention est de fournir un analyseur très bien adapté aux fluides comportant au moins une substance inflammable et qui offre un coût de fonctionnement plus compétitif.

A cet effet, l'invention a pour objet un analyseur du type décrit ci-dessus, dans lequel :
- la source de gaz est conçue pour délivrer un flux de gaz diluteur contenant un comburant de la substance inflammable, de préférence pour délivrer un flux d'air,
- l'injecteur est configuré pour introduire dans le flux de gaz diluteur des échantillons du fluide tels que la fraction volumique moyenne du fluide dans le flux gazeux est inférieure à 1/2000, de préférence inférieure à 1/20000, et
- le détecteur comprend au moins un micro-capteur pour détecter la substance à analyser.

Par « analyser le fluide», on entend au choix le fait de mesurer un ou des paramètres représentatifs de la fraction massique de la substance à analyser dans le fluide, ou bien simplement le fait de détecter la présence dans le fluide de la substance à analyser.

Par « A est connecté fluidiquement à B», on entend qu'il existe une connexion entre les éléments A et B permettant d'acheminer un fluide, par exemple une canalisation ou un tube capillaire. La connexion peut comprendre des éléments du type vanne de réglage, pompe, capteurs, ou des dérivations.

Par « fraction volumique moyenne du fluide dans le flux gazeux », on entend la fraction du fluide dans le flux gazeux en moyenne dans le temps, en tenant compte du fait que les échantillons de fluide sont injectés de manière discrète. Il s'agit donc du volume des échantillons injectés divisé par le volume du flux gazeux injecté pendant une certaine durée, suffisante pour que la moyenne obtenue ne dépendent pas de la durée choisie. Si l'injection d'échantillons est cyclique, la durée correspond à un nombre entier de périodes du cycle d'injection.

La substance à analyser peut être la substance inflammable en question, ou peut être une autre substance. Il peut y avoir plusieurs substances inflammables dans le fluide à analyser.

Selon des modes particuliers de réalisation, l'analyseur peut comprendre l'une ou plusieurs des caractéristiques suivantes, prise(s) isolément ou selon toutes les combinaisons techniquement possibles :
- l'injecteur est apte à introduire, de préférence cycliquement, dans le flux de gaz diluteur des échantillons de fluide ayant chacun une masse inférieure à 10 nanogrammes, de préférence inférieure à 1,5 nanogramme ;
- le détecteur est situé dans un boîtier ;
- l'analyseur comprend des moyens pour injecter un flux de gaz de balayage dans le boîtier, le boîtier comportant une sortie d'évacuation pour évacuer le flux de gaz de balayage, les moyens d'injection du flux de gaz de balayage et la sortie d'évacuation étant aptes à maintenir une surpression à l'intérieur du boîtier par rapport à l'extérieur du boîtier ;
- les moyens pour injecter le flux de gaz de balayage sont connectés fluidiquement à la source de gaz de manière à injecter du gaz diluteur comme gaz de balayage ;
- les moyens pour injecter le flux de gaz de balayage sont configurés pour injecter le flux de gaz de balayage dans le boîtier avec un débit supérieur à 5 fois un débit moyen du flux gazeux, de préférence supérieur ou égal à 9 fois un débit moyen du flux gazeux ;
- l'analyseur comprend une interface modulaire sur laquelle sont fixés le boîtier et l'injecteur, l'interface modulaire étant destinée à recevoir en entrée le fluide pour acheminer le fluide vers l'injecteur, et connectée à la source de gaz pour, au choix, acheminer le flux de gaz de balayage jusqu'au boîtier et/ou acheminer le flux de gaz diluteur jusqu'à l'injecteur ;
- le détecteur comporte une sortie pour libérer après analyse au moins une fraction, de préférence la totalité, du flux gazeux à l'intérieur du boîtier ;
- le détecteur comprend une carte électronique de commande, le micro-capteur (et la carte électronique comprenant chacun un encapsulage pour prévenir l'apparition d'une étincelle à l'intérieur du boîtier ;
- la source de gaz comprend un purificateur.

L'invention concerne aussi un procédé pour analyser un fluide comportant au moins une substance à analyser et au moins une substance inflammable, le procédé comprenant au moins les étapes suivantes :
a) obtention d'un flux de gaz diluteur à partir d'une source de gaz,
b) introduction dans le flux de gaz diluteur d'échantillons du fluide pour produire un flux gazeux, et
c) analyse du flux gazeux dans un détecteur,
le procédé étant caractérisé en ce que :
- à l'étape a), le flux de gaz diluteur comprend un comburant de la substance inflammable, et est de préférence un flux d'air,
- à l'étape b), les échantillons sont tels que la fraction volumique moyenne du fluide dans le flux gazeux est inférieure à 1/2000, de préférence inférieure à 1/20000, et
- à l'étape c), le détecteur au moins un micro-capteur pour détecter la substance à analyser.

Selon des modes particuliers de réalisation, le procédé peut comprendre l'une ou plusieurs des caractéristiques suivantes, prise(s) isolément ou selon toutes les combinaisons techniquement possibles :
- à l'étape b), les échantillons de fluide introduits dans le flux de gaz diluteur ont chacun une masse inférieure à 10 nanogrammes, de préférence inférieure à 1,5 nanogrammes ;
- à l'étape c), le détecteur est situé dans un boîtier dans lequel on injecte un flux de gaz de balayage, le flux de gaz de balayage s'échappant du boîtier par au moins une sortie d'évacuation, l'injection du flux de gaz de balayage maintenant une surpression à l'intérieur du boîtier par rapport à l'extérieur du boîtier ;
- à l'étape c), le boîtier présentant un volume interne, l'injection du flux de gaz de balayage est réalisée à un débit et à une température tels qu'aucune paroi délimitant le volume interne du boîtier ne comporte un point présentant une température supérieure ou égale à 85°C.

L'invention sera mieux comprise à la lecture de la description qui va suivre, donnée uniquement à titre d'exemple, et faite en se référant aux dessins annexés, sur lesquels :
- la figure 1 représente schématiquement un dispositif selon l'invention, l'intérieur du boîtier n'étant pas détaillé,
- la figure 2 représente schématiquement, en perspective, une partie du dispositif représenté sur la figure 1,
- la figure 3 représente schématiquement une vue de face, partiellement éclatée, de la partie du dispositif représentée sur la figure 2,
- la figure 4 représente schématiquement, en coupe transversale, la partie du dispositif représentée sur les figures 2 et 3,
- la figure 5 représente schématiquement, en vue de derrière, partiellement éclatée, de la partie du dispositif représentée sur les figures 2 et 3.

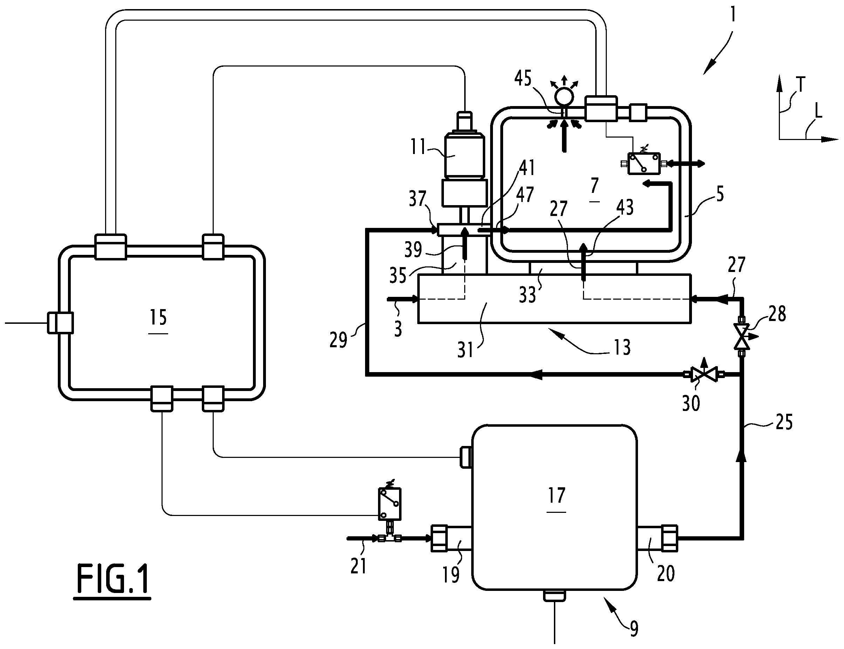

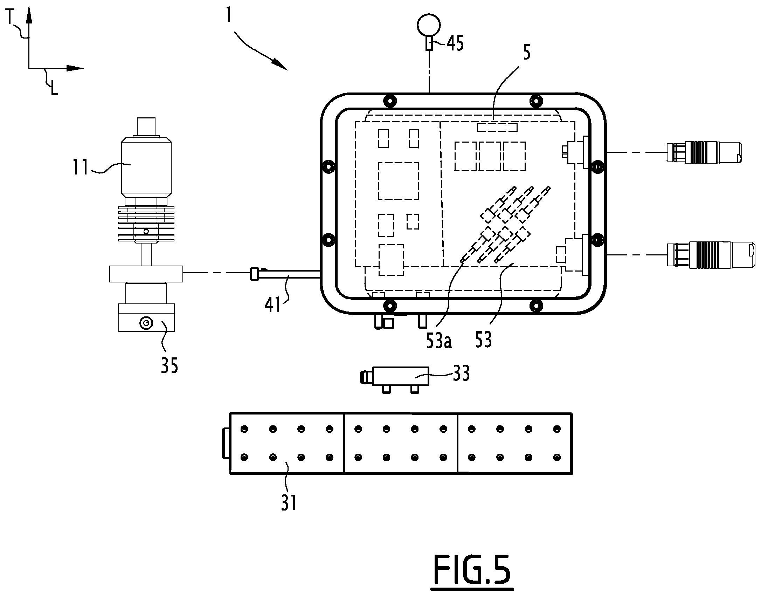

En référence aux figures 1 à 5, on décrit un analyseur 1 selon l'invention. L'analyseur 1 est par exemple destiné à analyser un fluide 3 provenant d'une raffinerie de pétrole.

Le fluide 3 est par exemple un mélange de gaz issu d'un procédé pétrochimique, le mélange de gaz comportant une substance à analyser, par exemple du benzène. Le benzène est une substance inflammable dont la LIE est de 1,2%.

L'analyseur 1 (figure 1) comprend un boîtier 5, un détecteur 7 situé dans le boîtier 5, une source d'air 9, et un injecteur 11 connecté fluidiquement en entrée à la source d'air 9 et recevant le fluide 3 à analyser, l'injecteur 11 étant connecté en sortie au détecteur 7 à travers le boîtier 5. L'analyseur 1 comporte avantageusement une interface modulaire 13 sur laquelle sont montés le boîtier 5 et l'injecteur 11.

L'analyseur 1 comprend en outre un ensemble 15 pour l'alimentation et le contrôle de la source d'air 9, de l'injecteur 11, et du détecteur 7.

La source de d'air 9 comprend avantageusement un purificateur 17 comportant une voie d'entrée 19 connectée à une ligne d'air instrument 21, et une voie de sortie 20 pour l'air purifié connectée à une canalisation d'air 25.

Le purificateur 17 est par exemple le générateur d'air Parker Balston 75-82EU, apte à retirer les hydrocarbures éventuellement présents dans l'air instrument 21 et à ramener leur concentration totale à moins de 0,1 ppm.

En variante, la source d'air peut être l'atmosphère, l'air atmosphérique étant de préférence filtré.

La canalisation d'air 25 comprend une première branche 27 pour conduire un flux d'air de balayage dans le boîtier 5 via l'interface modulaire 13. La première branche 27 comprend une vanne 28 de réglage du débit du flux d'air de balayage.

Par « flux de gaz », on entend un déplacement de ce gaz par convection forcée et auquel on peut associer un débit, qui caractérise l'intensité du flux.

La canalisation d'air 25 comprend une seconde branche 29 pour conduire un flux de gaz diluteur vers l'injecteur 11. La seconde branche 29 comprend une vanne 30 de réglage du débit du flux gaz diluteur.

L'interface modulaire 13 comprend plusieurs entrées et plusieurs sorties et permet de raccorder fluidiquement chaque entrée à une ou plusieurs sorties.

L'interface modulaire 13 est avantageusement analogue à l'ensemble décrit dans la demande WO-A-2007110504.

L'interface modulaire 13 est parfois dite « Nessi », c'est-à-dire conforme au standard ISA SP 76 et permettant l'interopérabilité entre différents composants provenant de différents constructeurs, et l'interchangeabilité desdits composants.

L'interface modulaire 13 comprend avantageusement un corps 31, s'étendant selon une direction longitudinale L, un module 33 de canalisation de l'air de balayage et de fixation du boîtier 5 sur le corps 31, et un module 35 de canalisation du fluide 3 et de fixation de l'injecteur 11 sur le corps 31.

Le corps 31 est analogue au corps (référencé 3) de l'ensemble décrit dans WO-A-2007110504.

Le module 33 est analogue à un module (référencé 5) de l'ensemble décrit dans WO-A-2007110504, le boîtier 5 de la présente invention jouant le rôle d'un des composants fonctionnels (référencé 7) de WO-A-2007110504.

Le module 35 est analogue à un autre module (référencé 5) de l'ensemble décrit dans WO-A-2007110504, l'injecteur 11 de la présente invention jouant le rôle d'un des composants fonctionnels 7 de WO-A-2007110504.

On définit également une direction transversale T perpendiculaire à la direction longitudinale L. La direction T est par exemple verticale, comme représenté sur les figures.

L'injecteur 11 s'étend par exemple selon la direction transversale T à partir de l'interface modulaire 13.

L'injecteur 11 comprend une première entrée 37 connectée fluidiquement à la seconde branche 29 pour recevoir le flux de gaz diluteur, une seconde entrée 39 connectée au module 35 pour recevoir le fluide 3, et une sortie 41 pour un flux gazeux résultant de l'introduction d'échantillons de fluide 3 dans le flux de gaz diluteur, la sortie 41 étant connectée fluidiquement au détecteur 7.

L'injecteur 11 est prévu pour introduire, par exemple cycliquement, une quantité calibrée de fluide 3 dans le gaz diluteur, avantageusement inférieure à 10 ng (nanogramme), de préférence inférieure à 1,5 ng.

L'injecteur 11 est avantageusement une vanne de type ROLSI™ décrite dans le document FR-A-2 853 414.

Le boîtier 5 forme un coffret répondant avantageusement à la prescription européenne ATEX, donc apte à protéger son contenu, en particulier le détecteur 7, d'explosions pouvant se produire autour du boîtier 5. Le coffret est par exemple apte à être pressurisé à une pression de 50 Pa au dessus de la pression régnant autour du boîtier 5.

Le boîtier 5 présente par exemple une forme générale sensiblement parallélépipédique. Le boîtier 5 s'étend par exemple selon la direction transversale T à partir de l'interface modulaire 13.

Le boîtier 5 comprend avantageusement une plaque interne 42 s'étendant par exemple sensiblement parallèlement à la direction longitudinale L et à la direction transversale T, et servant de support au détecteur 7.

Le boîtier 5 comprend une entrée 43 pour le gaz de balayage, un évent 45 pour évacuer les gaz contenus dans le boîtier 5, et une entrée 47 pour le flux gazeux destiné au détecteur 7.

L'évent 45 est apte à maintenir dans le boîtier 5 une surpression, avantageusement comprise entre 50 et 200 Pa. Par exemple, l'évent 45 assure une purge permanente et contrôlée pour maintenir une perte de charge entre l'intérieur du boîtier 5 et l'extérieur du boîtier 5 égale à la surpression désirée, par exemple égale à 50 Pa.

L'évent 45 comprend avantageusement un système d'arrêt de flamme pour éviter la propagation d'une combustion depuis l'extérieur du boîtier 5 vers l'intérieur du boîtier 5.

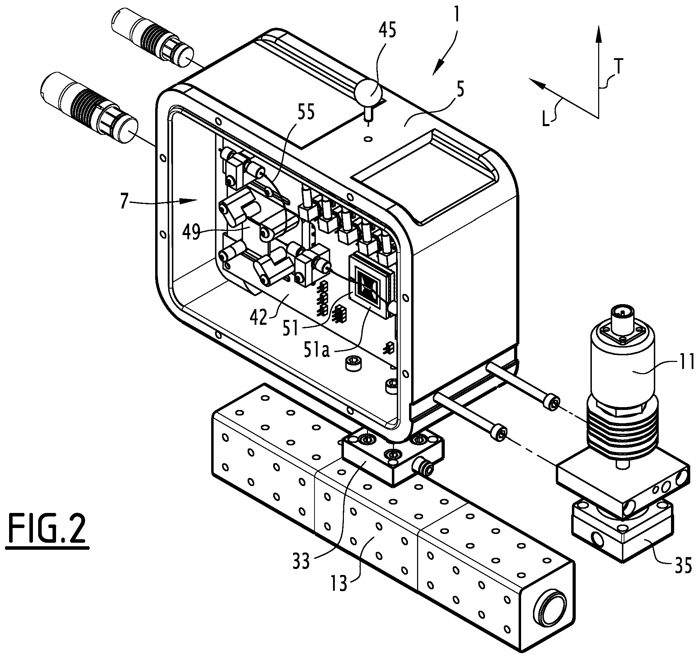

Le détecteur 7 (figures 2, 3 et 4) s'étend sur les deux faces de la plaque 42. Le détecteur 7 comprend une micro-colonne chromatographique 49, un micro-capteur 51 et une carte électronique 53.

La micro-colonne chromatographique 49 est par exemple du type gravé sur silicium. La micro-colonne chromatographique 49 est par exemple analogue à celle décrite dans le document WO-A-2011/154362, en particulier pages 11, ligne 16, à page 12, ligne 20. La micro-colonne chromatographique 49 est du type microcapillaire, avec un diamètre compris, par exemple, entre 10 µm et quelques centaines de µm. La micro-colonne chromatographique 49 présente par exemple une longueur à l'état « déplié » comprise entre 0,5 m et quelques mètres. La micro-colonne chromatographique 49 est revêtue intérieurement d'un film de matériau appelé « phase stationnaire ».

Le film est déposé selon des méthodes connues de l'homme du métier. Le film est par exemple un polymère, tel que le polyéthylène-glycol ou le polydiméthylsiloxane. Le film est un solide, un gel ou un liquide.

La micro-colonne chromatographique 49 est avantageusement fixée sur la plaque 42. En entrée, la micro-colonne chromatographique 49 est reliée par un tube capillaire 55 à l'entrée 47 du boîtier 5 pour recevoir le flux gazeux à analyser en provenance de la sortie 41 de l'injecteur 11. En sortie, la micro-colonne chromatographique 49 est reliée par un tube capillaire 57 au micro-capteur 51 pour envoyer le flux gazeux au micro-capteur 51.

Le micro-capteur 51 est par exemple de type NEMS (en anglais : *nano electro-mechanical system,* i. e. nano-système électromécanique). Il est par exemple du type de celui décrit dans le document WO-A-2011/154362, en particulier pages 12 à 15. Dans l'exemple représenté, le micro-capteur 51 est situé en sortie de la micro-colonne chromatographique 49. Le micro-capteur 51 est avantageusement fixé sur la plaque 42 du même côté que la micro-colonne chromatographique 49.

Le micro-capteur 51 comprend avantageusement au moins un microcapteur de la substance à analyser. Par « microcapteur », on entend un capteur dont la surface utile est d'environ 100 nm² à quelques µm².

Le micro-capteur 51 est par exemple du type gravimétrique. Il possède une surface vibrante apte à détecter et quantifier des molécules de la substance à analyser.

Le micro-capteur 51 est par exemple du type de ceux décrits dans Whiting, J.J., C.S. Fix, J.M. Anderson, et al. "High-speed two-dimensional gas chromatography using microfabricated GC columns combined with nanoelectromechanical mass sensors" dans TRANSDUCERS 2009 - 15th International Conference on Solid-State Sensors, Actuators and Microsystems, 2009.

Le micro-capteur 51 comprend avantageusement un encapsulage 51a apte à prévenir l'apparition d'une étincelle se développant à l'intérieur du boîtier 5 à partir du micro-capteur 51. L'encapsulage 51a répond par exemple à la norme EN 60079, partie 18.

Selon une variante non représentée, le micro-capteur 51 peut être situé à l'intérieur de la micro-colonne chromatographique 49. Selon une autre variante non représentée, il peut y avoir plusieurs micro-capteurs 51 distincts, situés au choix en sortie et/ou en différents points de la micro-colonne chromatographique 49, avantageusement sur une paroi interne de la micro-colonne chromatographique 49. Selon une autre variante avantageuse, un réseau de micro-capteurs 51 peut être disposé depuis l'entrée de la micro-colonne chromatographique 49 jusqu'à sa sortie.

Un tube capillaire 59 est connecté à une sortie du micro-capteur 51 pour évacuer le flux gazeux à l'intérieur du boîtier 5 via un diffuseur 60.

La carte électronique 53 est avantageusement fixée sur l'autre côté de la plaque 42 par rapport à la micro-colonne chromatographique 49 et au micro-capteur 51. La carte électronique 53 est connectée électriquement à la micro-colonne chromatographique 49 et au micro-capteur 51 pour alimenter et contrôler la micro-colonne chromatographique 49 et le micro-capteur 51, et pour acquérir des signaux électriques de mesure en provenance du micro-capteur 51.

La carte électronique 53 comprend avantageusement un encapsulage 53a apte à prévenir l'apparition d'une étincelle se développant à l'intérieur du boîtier 5 à partir de la carte électronique 53. L'encapsulage 53a répond par exemple à la norme EN 60079, partie 18.

L'ensemble 15 comprend des automatismes et asservissements connus en eux-mêmes de l'homme du métier.

On va maintenant décrire le fonctionnement de l'analyseur 1.

L'air instrument 21 (figure 1) entre dans le purificateur 17 par l'entrée 19. Le purificateur 17 est alimenté électriquement et contrôlé par l'ensemble 15. Le purificateur 17 purifie l'air instrument et envoie un flux d'air purifié dans la canalisation d'air 25.

Le flux d'air purifié se divise un flux d'air de balayage dans la première branche 27 et un flux de gaz diluteur dans la branche 29. Les vannes de réglages 28, 30 permettent de régler sélectivement les débits du flux d'air de balayage et du flux de gaz diluteur.

Avantageusement, le débit du flux de gaz diluteur est compris entre 0,1 et 3 ml/min. Il est par exemple d'environ 1 ml/minute. Le flux de gaz diluteur est, avant son entrée dans le boîtier 5, par exemple à une pression d'environ 50 Pa au dessus de la pression ambiante et par exemple à la température ambiante.

Le débit du flux d'air de balayage dans le boîtier 5 est avantageusement supérieur à 5 fois le débit du flux de gaz diluteur. Par exemple il est d'environ 9 fois celui du flux de gaz diluteur, soit environ 9 ml/minute. Le flux d'air de balayage est, avant son entrée dans le boîtier 5, par exemple à une pression d'environ 50 Pa au dessus de la pression ambiante et par exemple à la température ambiante.

Le flux d'air de balayage passe dans le corps 31 de l'interface modulaire 13, puis dans le module 33 et entre dans le boîtier 5 par l'entrée 43. Le flux d'air de balayage balaie ensuite l'intérieur du boîtier 5 pour ressortir par l'évent 45. Le balayage a avantageusement lieu des deux côtés de la plaque 42.

Avantageusement, l'injection du flux de gaz de balayage est réalisée à un débit et à une température tels qu'aucune paroi délimitant le volume interne du boîtier 5 ne comporte un point présentant une température supérieure ou égale à 85°C, de manière à réduire les risques d'inflammation des gaz présents à l'intérieur du boîtier 5.

Le flux de gaz diluteur arrive à la première entrée 37 de l'injecteur 11.

Le fluide à analyser 3 passe dans le corps 31 l'interface modulaire 13, puis une partie du fluide 3 arrive à la seconde entrée 39 de l'injecteur 11 via le module 35.

L'injecteur 11 est alimenté électriquement et contrôlé par l'ensemble 15. L'injecteur 11 introduit des échantillons du fluide 3 arrivant par la seconde entrée 39 dans le gaz diluteur arrivant par la première entrée 37 et produit un flux gazeux sortant par la sortie 41.

L'injecteur 11 réalise une dilution du fluide 3 par le gaz diluteur. La dilution amène la fraction volumique de tous les constituants du flux gazeux provenant du fluide 3 en dessous de 1/2000, de préférence 1/20000. Dit autrement, la dilution amène la fraction volumique de tous les constituants du flux gazeux provenant du fluide 3 au moins un ordre de grandeur, de préférence deux ordres de grandeur, en dessous de 0,5%, i. e. des LIE des substances inflammables les plus contraignantes.

Par exemple, l'injecteur 11 introduit une fois par minute un échantillon de masse avantageusement inférieure à 10 ng, par exemple de 1 ng, dans le flux de gaz diluteur, ce qui assure que la fraction volumique moyenne du fluide 3 dans le flux gazeux est inférieure à 1/2000.

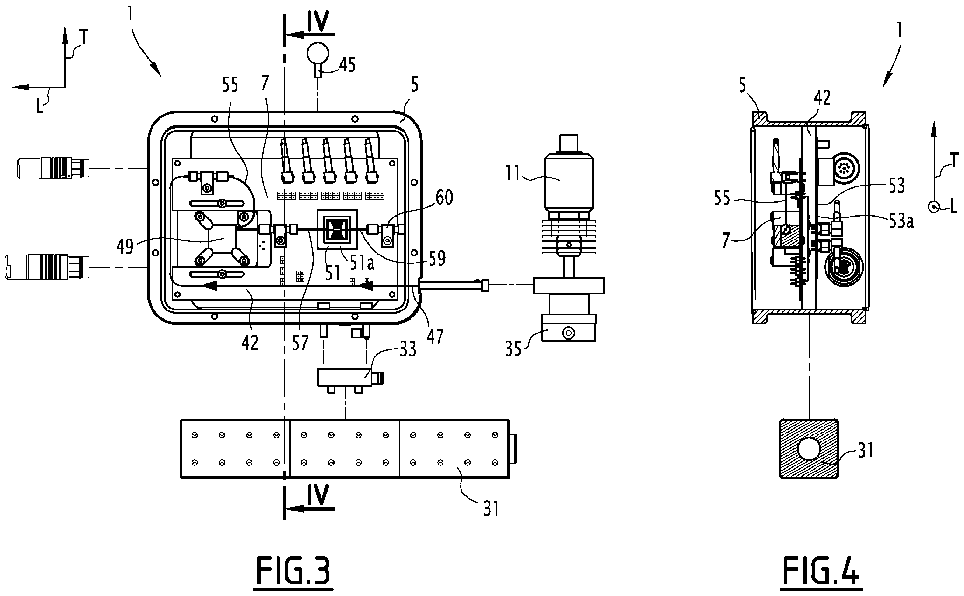

Le flux gazeux qui comprend le fluide 3 ainsi dilué entre dans le boîtier 5 par l'entrée 47 (figure 3) et arrive par le tube capillaire 55 à l'entrée de la micro-colonne chromatographique 49. La micro-colonne chromatographique 49 sépare les substances contenues dans le flux gazeux par migration différentielle en fonction de leurs affinités respectives avec la phase stationnaire, chaque substance possédant une vitesse de migration respective qui dépend de son affinité avec la phase stationnaire.

Le flux gazeux sort ensuite de la micro-colonne chromatographique 49 par le tube capillaire 57 et arrive dans le micro-capteur 51.

Le micro-capteur 51 détecte la présence de la substance à analyser et/ou mesure la quantité de substance à analyser dans le flux gazeux qui le traverse.

Le micro-capteur 51 mesure une variation des paramètres qui caractérisent la vibration de la surface vibrante, par exemple la fréquence de résonance de la surface vibrante.

Pour créer cette résonance, la carte électronique 53 excite le micro-capteur 51 à une fréquence particulière. La carte électronique 53 mesure des signaux électriques créés par cette résonance et les traite ou les envoie à un terminal distant. Les signaux du micro-capteur 51 sont en général analogiques, de faible niveau (de l'ordre du mV), et à haute fréquence (de une dizaine de mégahertz à quelques centaines de mégahertz).

Pour minimiser la perturbation de ces signaux par des champs électromagnétiques extérieurs, il est utile de minimiser la longueur des connexions électriques entre le micro-capteur 51 et la carte électronique 53. Il est donc avantageux de localiser la carte électronique 53 dans le boîtier 5.

Le flux gazeux, après son passage dans le micro-capteur 51, emprunte le tube capillaire 59 est relâché dans le boîtier 5 via l'élément 60. Le flux gazeux se mélange alors au le flux d'air de balayage, ce qui crée une nouvelle dilution d'environ un ordre de grandeur des substances inflammables contenues dans le flux gazeux.

Grâce aux caractéristiques de l'analyseur 1 décrit ci-dessus, le fluide 3, bien que contenant au moins une substance inflammable, est mis en contact avec de l'air en tant que gaz diluteur, sans risque d'explosion, du fait d'une dilution amenant la fraction volumique moyenne de toutes les substances contenues dans le flux gazeux à des valeurs inférieures de un à deux ordres de grandeur aux LIE les plus contraignantes. Une telle dilution est possible du fait que le détecteur 7 comprend une micro-colonne chromatographique 49 et un micro-capteur 51 permettant la séparation et la détection de la substance à analyser avec une concentration très faible.

Ainsi, le détecteur 7 parvient à analyser la substance à analyser malgré sa dilution très importante. L'analyseur 1 est donc très bien adapté aux fluides comportant au moins une substance inflammable, tels que le fluide 3. Comme l'analyseur 1 utilise de l'air comme gaz diluteur, son coût de fonctionnement est plus compétitif que s'il utilisait un gaz spécial, tel que de l'azote.

Le micro-capteur 51 étant de type NEMS et la micro-colonne chromatographique 49 étant gravée sur du silicium, il est possible d'introduire dans le flux de gaz diluteur des échantillons de fluide 3 de masse réduite, telle qu'environ 1 ng, ce qui réduit encore le risque d'explosion du flux gazeux envoyé au détecteur 7.

De plus, la caractéristique optionnelle selon laquelle on réalise un balayage à l'air dans le boîtier 5, le flux gazeux se mélangeant au flux d'air de balayage après analyse dans le détecteur 7, permet de diluer encore davantage les substances inflammables dans le boîtier 5.

La dilution du fluide 3 dans le flux gazeux présente en outre l'avantage que, si des substances toxiques sont présentes dans le fluide 3, des quantités très faibles de ces substances toxiques sont émises par le dispositif 1.

Selon une variante non représentée, l'injecteur 11 et/ou le purificateur d'air 17 peuvent être situés à l'intérieur du boîtier 5, de manière à bénéficier de la protection contre les atmosphères explosives conférée par le boîtier 5.

## Revendications

1. Analyseur (1) pour analyser un fluide (3) comportant au moins une substance à analyser et au moins une substance inflammable, l'analyseur (1) comprenant :
- une source de gaz (9) pour fournir un flux de gaz diluteur,
- un injecteur (11) pour introduire des échantillons du fluide (3) dans le flux de gaz diluteur et produire un flux gazeux, et
- un détecteur (7) pour analyser le flux gazeux, le détecteur (7) comprenant au moins un micro-capteur (51) pour détecter la substance à analyser, l'analyseur (1) étant **caractérisé en ce que** :
- la source de gaz (9) est conçue pour délivrer un flux de gaz diluteur contenant un comburant de la substance inflammable, de préférence pour délivrer un flux d'air,
- l'injecteur (11) est configuré pour introduire dans le flux de gaz diluteur des échantillons du fluide (3) tels que la fraction volumique moyenne du fluide (3) dans le flux gazeux est inférieure à 1/2000, de préférence inférieure à 1/20000.

2. Analyseur (1) selon la revendication 1, **caractérisé en ce que** l'injecteur (11) est apte à introduire, de préférence cycliquement, dans le flux de gaz diluteur des échantillons de fluide (3) ayant chacun une masse inférieure à 10 nanogrammes, de préférence inférieure à 1,5 nanogramme.

3. Analyseur (1) selon la revendication 1 ou 2, **caractérisé en ce que** le détecteur (7) est situé dans un boîtier (5).

4. Analyseur (1) selon la revendication 3, **caractérisé en ce qu'**il comprend des moyens (21, 17, 25, 27, 43) pour injecter un flux de gaz de balayage dans le boîtier (5), le boîtier (5) comportant une sortie d'évacuation (45) pour évacuer le flux de gaz de balayage, les moyens (21, 17, 25, 27, 43) d'injection du flux de gaz de balayage et la sortie d'évacuation (45) étant aptes à maintenir une surpression à l'intérieur du boîtier (5) par rapport à l'extérieur du boîtier (5).

5. Analyseur (1) selon la revendication 4, **caractérisé en ce que** les moyens pour injecter le flux de gaz de balayage (21, 17, 25, 27, 43) sont connectés fluidiquement à la source de gaz (9) de manière à injecter du gaz diluteur comme gaz de balayage.

6. Analyseur (1) selon la revendications 4 ou 5, **caractérisé en ce que** les moyens (21, 17, 25, 27, 43) pour injecter le flux de gaz de balayage sont configurés pour injecter le flux de gaz de balayage dans le boîtier (5) avec un débit supérieur à 5 fois un débit moyen du flux gazeux, de préférence supérieur ou égal à 9 fois un débit moyen du flux gazeux.

7. Analyseur (1) selon l'une quelconque des revendications 4 à 6, **caractérisé en ce qu'**il comprend une interface modulaire (13) sur laquelle sont fixés le boîtier (5) et l'injecteur (11), l'interface modulaire (13) étant :
- destinée à recevoir en entrée le fluide (3) pour acheminer le fluide (3) vers l'injecteur (11), et
- connectée à la source de gaz (9) pour, au choix, acheminer le flux de gaz de balayage jusqu'au boîtier (5) et/ou acheminer le flux de gaz diluteur jusqu'à l'injecteur (11).

8. Analyseur (1) selon l'une quelconque des revendications 3 à 7, **caractérisé en ce que** le détecteur (7) comporte une sortie (59-60) pour libérer après analyse au moins une fraction, de préférence la totalité, du flux gazeux à l'intérieur du boîtier (5).

9. Analyseur (1) selon l'une quelconque des revendications 3 à 8, **caractérisé en ce que** le détecteur (7) comprend une carte électronique (53) de commande, le micro-capteur (51) et la carte électronique (53) comprenant chacun un encapsulage (51a, 53a) pour prévenir l'apparition d'une étincelle à l'intérieur du boîtier (5).

10. Analyseur (1) selon l'une quelconque des revendications 1 à 9, **caractérisé en ce que** la source de gaz (9) comprend un purificateur (17).

11. Procédé pour analyser un fluide (3) comportant au moins une substance à analyser et au moins une substance inflammable, le procédé comprenant au moins les étapes suivantes :
a) obtention d'un flux de gaz diluteur à partir d'une source de gaz (9),
b) introduction dans le flux de gaz diluteur d'échantillons du fluide (3) pour produire un flux gazeux, et
c) analyse du flux gazeux dans un détecteur (7), le détecteur (7) comprenant au moins un micro-capteur (51) pour détecter la substance à analyser,
le procédé étant **caractérisé en ce que** :
- à l'étape a), le flux de gaz diluteur comprend un comburant de la substance inflammable, et est de préférence un flux d'air,
- à l'étape b), les échantillons sont tels que la fraction volumique moyenne du fluide (3) dans le flux gazeux est inférieure à 1/2000, de préférence inférieure à 1/20000.

12. Procédé selon la revendication 11, dans lequel, à l'étape b), les échantillons de fluide (3) introduits dans le flux de gaz diluteur ont chacun une masse inférieure à 10 nanogrammes, de préférence inférieure à 1,5 nanogrammes.

13. Procédé selon la revendication 11 ou 12, **caractérisé en ce que**, à l'étape c), le détecteur (7) est situé dans un boîtier (5) dans lequel on injecte un flux de gaz de balayage, le flux de gaz de balayage s'échappant du boîtier (5) par au moins une sortie d'évacuation (45), l'injection du flux de gaz de balayage maintenant une surpression à l'intérieur du boîtier (5) par rapport à l'extérieur du boîtier (5).

14. Procédé selon la revendication 13, **caractérisé en ce que**, à l'étape c), le boîtier (5) présentant un volume interne, l'injection du flux de gaz de balayage est réalisée à un débit et à une température tels qu'aucune paroi délimitant le volume interne du boîtier (5) ne comporte un point présentant une température supérieure ou égale à 85°C.
1. Analyseur (1) pour analyser un fluide (3) comportant au moins une substance à analyser et au moins une substance inflammable, l'analyseur (1) comprenant :
- une source de gaz (9) pour fournir un flux de gaz diluteur,
- un injecteur (11) pour introduire des échantillons du fluide (3) dans le flux de gaz diluteur et produire un flux gazeux, et
- un détecteur (7) pour analyser le flux gazeux,
l'analyseur (1) étant **caractérisé en ce que** :
- la source de gaz (9) est conçue pour délivrer un flux de gaz diluteur contenant un comburant de la substance inflammable, de préférence pour délivrer un flux d'air,
- l'injecteur (11) est configuré pour introduire dans le flux de gaz diluteur des échantillons du fluide (3) tels que la fraction volumique moyenne du fluide (3) dans le flux gazeux est inférieure à 1/2000, de préférence inférieure à 1/20000, et
- le détecteur (7) comprend au moins un micro-capteur (51) pour détecter la substance à analyser.
2. Analyseur (1) selon la revendication 1, **caractérisé en ce que** l'injecteur (11) est apte à introduire, de préférence cycliquement, dans le flux de gaz diluteur des échantillons de fluide (3) ayant chacun une masse inférieure à 10 nanogrammes, de préférence inférieure à 1,5 nanogramme.
3. Analyseur (1) selon la revendication 1 ou 2, **caractérisé en ce que** le détecteur (7) est situé dans un boîtier (5).
4. Analyseur (1) selon la revendication 3, **caractérisé en ce qu'**il comprend des moyens (21, 17, 25, 27, 43) pour injecter un flux de gaz de balayage dans le boîtier (5), le boîtier (5) comportant une sortie d'évacuation (45) pour évacuer le flux de gaz de balayage, les moyens (21, 17, 25, 27, 43) d'injection du flux de gaz de balayage et la sortie d'évacuation (45) étant aptes à maintenir une surpression à l'intérieur du boîtier (5) par rapport à l'extérieur du boîtier (5).
5. Analyseur (1) selon la revendication 4, **caractérisé en ce que** les moyens pour injecter le flux de gaz de balayage (21, 17, 25, 27, 43) sont connectés fluidiquement à la source de gaz (9) de manière à injecter du gaz diluteur comme gaz de balayage.
6. Analyseur (1) selon la revendications 4 ou 5, **caractérisé en ce que** les moyens (21, 17, 25, 27, 43) pour injecter le flux de gaz de balayage sont configurés pour injecter le flux de gaz de balayage dans le boîtier (5) avec un débit supérieur à 5 fois un débit moyen du flux gazeux, de préférence supérieur ou égal à 9 fois un débit moyen du flux gazeux.
7. Analyseur (1) selon l'une quelconque des revendications 4 à 6, **caractérisé en ce qu'**il comprend une interface modulaire (13) sur laquelle sont fixés le boîtier (5) et l'injecteur (11), l'interface modulaire (13) étant :
- destinée à recevoir en entrée le fluide (3) pour acheminer le fluide (3) vers l'injecteur (11), et
- connectée à la source de gaz (9) pour, au choix, acheminer le flux de gaz de balayage jusqu'au boîtier (5) et/ou acheminer le flux de gaz diluteur jusqu'à l'injecteur (11).
8. Analyseur (1) selon l'une quelconque des revendications 3 à 7, **caractérisé en ce que** le détecteur (7) comporte une sortie (59-60) pour libérer après analyse au moins une fraction, de préférence la totalité, du flux gazeux à l'intérieur du boîtier (5).
9. Analyseur (1) selon l'une quelconque des revendications 3 à 8, **caractérisé en ce que** le détecteur (7) comprend une carte électronique (53) de commande, le micro-capteur (51) et la carte électronique (53) comprenant chacun un encapsulage (51a, 53a) pour prévenir l'apparition d'une étincelle à l'intérieur du boîtier (5).
10. Analyseur (1) selon l'une quelconque des revendications 1 à 9, **caractérisé en ce que** la source de gaz (9) comprend un purificateur (17).
11. Procédé pour analyser un fluide (3) comportant au moins une substance à analyser et au moins une substance inflammable, le procédé comprenant au moins les étapes suivantes :
a) obtention d'un flux de gaz diluteur à partir d'une source de gaz (9),
b) introduction dans le flux de gaz diluteur d'échantillons du fluide (3) pour produire un flux gazeux, et
c) analyse du flux gazeux dans un détecteur (7),
le procédé étant **caractérisé en ce que** :
- à l'étape a), le flux de gaz diluteur comprend un comburant de la substance inflammable, et est de préférence un flux d'air,
- à l'étape b), les échantillons sont tels que la fraction volumique moyenne du fluide (3) dans le flux gazeux est inférieure à 1/2000, de préférence inférieure à 1/20000, et
- à l'étape c), le détecteur (7) au moins un micro-capteur (51) pour détecter la substance à analyser.
12. Procédé selon la revendication 11, dans lequel, à l'étape b), les échantillons de fluide (3) introduits dans le flux de gaz diluteur ont chacun une masse inférieure à 10 nanogrammes, de préférence inférieure à 1,5 nanogrammes.
13. Procédé selon la revendication 11 ou 12, **caractérisé en ce que**, à l'étape c), le détecteur (7) est situé dans un boîtier (5) dans lequel on injecte un flux de gaz de balayage, le flux de gaz de balayage s'échappant du boîtier (5) par au moins une sortie d'évacuation (45), l'injection du flux de gaz de balayage maintenant une surpression à l'intérieur du boîtier (5) par rapport à l'extérieur du boîtier (5).
14. Procédé selon la revendication 13, **caractérisé en ce que**, à l'étape c), le boîtier (5) présentant un volume interne, l'injection du flux de gaz de balayage est réalisée à un débit et à une température tels qu'aucune paroi délimitant le volume interne du boîtier (5) ne comporte un point présentant une température supérieure ou égale à 85°C.

## Patentansprüche

1. Analysevorrichtung (1) zum Analysieren eines Fluid (3), welches wenigstens eine zu analysierende Substanz und wenigstens eine entzündliche Substanz aufweist, wobei die Analysevorrichtung (1) aufweist:
- eine Gasquelle (9) zum Liefern eines Verdünnungsgasstroms,
- eine Einspritzvorrichtung (11) zum Einbringen von Proben des Fluid (3) in den Verdünnungsgasstrom und Produzieren eines Gasstroms, und
- einen Detektor (7) zum Analysieren des Gasstroms,
wobei die Analysevorrichtung (1) **dadurch gekennzeichnet ist, dass**
- die Gasquelle (9) eingerichtet ist zum Liefern eines Verdünnungsgasstroms, der einen Verbrennungsförderer der entzündlichen Substanz enthält, bevorzugt zum Liefern eines Luftstroms,
- die Einspritzvorrichtung (11) konfiguriert ist zum Einbringen der Proben des Fluid (3) in den Verdünnungsgasstrom, die so sind, dass die mittlere Volumenfraktion des Fluid (3) in dem Gasstrom kleiner als 1/2000, bevorzugt kleiner als 1/20000 ist, und
- der Detektor (7) wenigstens einen Mikro-Sensor (51) zum Detektieren der zu analysierenden Substanz aufweist.

2. Analysevorrichtung (1) gemäß Anspruch 1, **dadurch gekennzeichnet, dass** die Einspritzvorrichtung (11) imstande ist, Proben des Fluid (3), die eine Masse kleiner als 10 Nanogramm, bevorzugt kleiner als 1,5 Nanogramm, haben, in den Verdünnungsgasstrom, bevorzugt zyklisch, einzubringen.

3. Analysevorrichtung (1) gemäß Anspruch 1 oder 2, **dadurch gekennzeichnet, dass** der Detektor (7) in einem Gehäuse (5) angeordnet ist.

4. Analysevorrichtung (1) gemäß Anspruch 3, **dadurch gekennzeichnet, dass** sie aufweist Mittel (21, 17, 25, 27, 43) zum Einspritzen eines Abtastgasstroms in das Gehäuse (5), wobei das Gehäuse (5) einen Evakuier-Ausgang (45) zum Evakuieren des Abtastgasstroms aufweist, wobei die Mittel (21, 17, 25, 27, 43) zum Einspritzen des Abtastgasstroms und der Evakuier-Ausgang (45) imstande sind, einen Überdruck im Inneren des Gehäuses (5) bezüglich des Äußeren des Gehäuses (5) aufrechtzuerhalten.

5. Analysevorrichtung (1) gemäß Anspruch 4, **dadurch gekennzeichnet, dass** die Mittel zum Einspritzen des Abtastgasstroms (21, 17, 25, 27, 43) mit der Gasquelle (9) fluidverbunden sind, um das Verdünnungsgas als Abtastgas einzuspritzen.

6. Analysevorrichtung (1) gemäß Anspruch 4 oder 5, **dadurch gekennzeichnet, dass** die Mittel (21, 17, 25, 27, 43) zum Einspritzen des Abtastgasstroms konfiguriert sind zum Einspritzen des Abtastgasstroms in das Gehäuse (5) mit einer Durchströmungsmenge, die 5-mal größer ist als eine mittlere Durchströmungsmenge des Gasstroms, bevorzugt größer oder gleich 9-mal einer mittleren Durchströmungsmenge des Gasstroms.

7. Analysevorrichtung (1) gemäß irgendeinem der Ansprüche 4 bis 6, **dadurch gekennzeichnet, dass** sie aufweist ein modulares Interface (13), an welchem das Gehäuse (5) und die Einspritzvorrichtung (11) befestigt sind, wobei das modulare Interface (13):
- dazu bestimmt ist, am Eingang das Fluid (3) zu empfangen zum Weiterleiten des Fluid (3) zu der Einspritzvorrichtung (11), und
- verbunden ist mit der Gasquelle (9) zum, wahlweise, Weiterleiten des Abtastgasstroms zu dem Gehäuse (5) und/oder Weiterleiten des Verdünnungsgasstroms zu der Einspritzvorrichtung (11).

8. Analysevorrichtung (1) gemäß irgendeinem der Ansprüche 3 bis 7, **dadurch gekennzeichnet, dass** der Detektor (7) aufweist einen Ausgang (59-60) zum nach der Analyse Freigeben wenigstens einer Fraktion, bevorzugt der Gesamtheit, des Gasstroms in das Innere des Gehäuses (5).

9. Analysevorrichtung (1) gemäß irgendeinem der Ansprüche 3 bis 8, **dadurch gekennzeichnet, dass** der Detektor (7) aufweist eine elektronische Steuerkarte (53), wobei der Mikro-Sensor (51) und die elektronische Karte (53) jeweils eine Einkapselung (51a, 53a) aufweisen zum Verhindern des Auftretens eines Zündfunkens im Inneren des Gehäuses (5).

10. Analysevorrichtung (1) gemäß irgendeinem der Ansprüche 1 bis 9, **dadurch gekennzeichnet, dass** die Gasquelle (9) eine Reinigungsvorrichtung (17) aufweist.

11. Verfahren zum Analysieren eines Fluid (3), das wenigstens eine zu analysierende Substanz und wenigstens eine entzündliche Substanz aufweist, wobei das Verfahren aufweist wenigstens die folgenden Schritte:
a) Erlangen eines Verdünnungsgasstroms ausgehend von einer Gasquelle (9),
b) Einbringen von Proben des Fluid (3) in den Verdünnungsgasstrom zum Produzieren eines Gasstroms, und
c) Analysieren des Gasstroms in einem Detektor (7),
wobei das Verfahren **dadurch gekennzeichnet ist, dass**:
- im Schritt a) der Verdünnungsgasstrom einen Verbrennungsförderer der entzündlichen Substanz aufweist, und bevorzugt ein Luftstrom ist,
- im Schritt (b) die Proben so sind, dass die mittlere Volumenfraktion des Fluid (3) in dem Gasstrom kleiner ist als 1/2000, bevorzugt kleiner als 1/20000, und
- im Schritt c) der Detektor (7) wenigstens ein Mikro-Sensor (51) zum Detektieren der zu analysieren Substanz.

12. Verfahren gemäß Anspruch 11, wobei im Schritt b) die Proben des Fluid (3), die in den Verdünnungsgasstrom eingebracht werden, jeweils eine Masse kleiner als 10 Nanogramm, bevorzugt kleiner als 1,5 Nanogramm, haben.

13. Verfahren gemäß Anspruch 11 oder 12, **dadurch gekennzeichnet, dass** im Schritt (c) der Detektor (7) in einem Gehäuse (5) angeordnet ist, in welches man einen Abtastgasstrom einspritzt, wobei der Abtastgasstrom aus dem Gehäuse entweicht mittels wenigstens eines Evakuier-Ausgangs (45), wobei das Einspritzen des Abtastgasstroms einen Überdruck im Inneren des Gehäuses (5) bezüglich des Äußeren des Gehäuses (5) aufrechterhält.

14. Verfahren gemäß Anspruch 13, **dadurch gekennzeichnet, dass** im Schritt c) das Gehäuse (5) ein Innenvolumen hat, wobei das Einspritzen des Abtastgasstroms realisiert wird mit einer Durchströmungsmenge und einer Temperatur, die so sind, dass keine Wand, die das Innenvolumen des Gehäuses (5) begrenzt, eine Stelle mit einer Temperatur größer oder gleich 85°C aufweist.

## Claims

1. An analyser (1) for analysing a fluid (3) containing at least one substance to be analysed and at least one inflammable substance, the analyser (1) containing:
- a source of gas (9) to provide a flux of diluent gas,
- an injecting nozzle (11) for introducing samples of the fluid (3) into the flux of diluent gas and producing a gaseous flux, and
- a detector (7) for analysing the gaseous flux, the detector (7) comprising at least one micro sensor (51) for detecting the substance to be analysed,
the analyser (1) being **characterized in that**:
- the source of gas (9) is intended to deliver a flux of diluent gas containing a substance supporting combustion of the inflammable substance, preferably to deliver a flux of air,
- the injecting nozzle (11) is configured to introduce into the flux of diluent gas samples of the fluid (3) such that the average volume fraction of the fluid (3) in the gaseous flux is below 1/2000 and preferably below 1/20000.

2. The analyser (1) according to claim 1, wherein the injecting nozzle (11) is capable of introducing into the flux of diluent gas, preferably in cyclical manner, samples of the fluid (3) each weighing less than 10 nanograms and preferably less than 1.5 nanogram.

3. The analyser (1) according to claim 1 or 2, wherein the detector (7) is located in a box (5).

4. The analyser (1) according to claim 3, wherein the analyser (1) contains means (21, 17, 25, 27, 43) for injecting a flux of purge gas into the box (5), the box (5) comprising an evacuation outlet (45) for evacuating the flux of purge gas, the means (21, 17, 25, 27, 43) for injecting the flux of purge gas and the evacuation outlet (45) being capable of maintaining inside the box (5) an overpressure relative to the outside of the box (5).

5. The analyser (1) according to claim 4, wherein the means for injecting the flux of purge gas (21, 17, 25, 27, 43) are connected fluidically to the source of gas (9) so as to inject the diluent gas as the purge gas.

6. The analyser (1) according to claim 4 or 5, wherein the means (21, 17, 25, 27, 43) for injecting the flux of purge gas are configured to inject the flux of purge gas into the box (5) with a flow rate higher than 5 times the average flow rate of the gaseous flux and preferably higher than or equal to 9 times the average flow rate of the gaseous flux.

7. The analyser (1) according to any one of claims 4 to 6, wherein the analyser (1) comprises a modular interface (13) on which the box (5) and the injecting nozzle (11) are fixed, the modular interface (13) being:
- intended to receive as intake the fluid (3) so as to route the fluid (3) toward the injection nozzle, (11) and
- connected to the source of gas (9) so as to route the flux of purge gas to the box (5) and/or to route the flux of diluent gas to the injection nozzle (11).

8. The analyser (1) according to any one of claims 3 to 7, wherein the detector (7) has an outlet (59-60) to set free, after analysis, at least a fraction and preferably the totality of the gaseous flux inside the box (5).

9. The analyser (1) according to any one of claims 3 to 8, wherein the detector (7) contains an electronic control card (53), the micro sensor (51) and the electronic card (53) each being provided with a packing (51a, 53a) to prevent the forming of a spark inside the box (5).

10. The analyser (1) according to any one of claims 1 to 9, wherein the source of gas (9) contains a purifier (17).

11. A method for analysing a fluid (3) containing at least one substance to be analysed and at least one inflammable substance, the method comprising at least the following steps:
a) obtaining a flux of diluent gas from a source of gas (9),
b) introducing into the flux of diluent gas samples of the fluid (3) to produce a gaseous flux, and
c) analysing the gaseous flux in a detector (7), the detector (7) comprising at least a micro sensor (51) for detecting the substance to be analysed,
the method being **characterized in that**:
- in step a) the flux of diluent gas contains a material capable of supporting the combustion of the inflammable substance, the flux of diluent gas preferably being a flux of air,
- in step b) the samples are such that the average volume fraction of the fluid (3) in the gaseous flux is less than 1/2000, preferably less than 1/20000.

12. The method according to claim 11, in which, in step b), each of the samples of the fluid (3) introduced into the flux of diluent gas weighs less than 10 nanograms and preferably less than 1.5 nanogram.

13. The method according to claim 11 or 12, wherein in step c) the detector (7) is located in a box (5) into which is injected a flux of purge gas, the purge gas leaving the box (5) through at least one evacuation outlet (45), the injection of the flux of purge gas maintaining an overpressure inside the box (5) relative to the outside of the box (5).

14. The method according to claim 13, wherein in step c), the box (5) presenting an internal volume, the injection of the purge gas takes place with a flow rate and temperature such that on no wall delimiting the internal volume of the box (5) there is a point presenting a temperature higher than or equal to 85°C.
